Europäisches Patentamt

European Patent Office

Office européen des brevets

(11) Veröffentlichungsnummer : **0 069 995**
**B1**

(12) **EUROPÄISCHE PATENTSCHRIFT**

(45) Veröffentlichungstag der Patentschrift :
07.05.86

(21) Anmeldenummer : 82106122.3

(22) Anmeldetag : 08.07.82

(51) Int. Cl.⁴ : **C 12 P   1/00, A 61 K 35/78**

(54) Verfahren zur Herstellung eines Bienenpollengemisches mit verbesserter Resorbierbarkeit sowie ein zur Hyposensibilisierung geeignetes Bienenpollengemisch.

(30) Priorität : 10.07.81 DE 3127354

(43) Veröffentlichungstag der Anmeldung :
19.01.83 Patentblatt 83/03

(45) Bekanntmachung des Hinweises auf die Patenterteilung : 07.05.86 Patentblatt 86/19

(84) Benannte Vertragsstaaten :
AT BE CH DE FR GB IT LI LU NL SE

(56) Entgegenhaltungen :
DE-A- 1 467 750
FR-A- 1 054 555
FR-A- 1 164 258
FR-A- 1 191 459
FR-A- 2 443 265

(73) Patentinhaber : Alsitan Gesellschaft Wilh. E. Ronneburg & Co.
Am Bühl 16-18
D-8919 Greifenberg (Ammersee) (DE)

(72) Erfinder : Ronneburg, Wilhelm E.
Am Bühl 16-18
D-8919 Greifenberg (Ammersee) (DE)

(74) Vertreter : Kolb, Helga, Dr. rer. nat. et al
Hoffmann, Eitle & Partner Patentanwälte Arabellastrasse 4
D-8000 München 81 (DE)

**Beschreibung**

Die Erfindung betrifft ein Verfahren zur Verbesserung der Resorbierbarkeit von als Heil- und Nahrungsmittel dienenden Bienenpollen.

Der von den Bienen gesammelten Pollen, der z. B. im mitteleuropäischen Raum aus Pollen von 43 bis 49 der wichtigsten Blütenfamilien bestehen kann, enthält wertvolle Substanzen, wie Eiweissstoffe, DNS, Zucker, Stärke, Fette, Carotinoide, Folsäure, Ascorbinsäure sowie zahlreiche noch nicht aufgeklärte Substanzen, die das Wachstum höherer Tiere merklich beschleunigen können. Die Einnahme dieser gesammelten Pollenkörner gilt seit langem für überbeanspruchte Menschen, Genesende, Sportler, aber auch für Kinder und Jugendliche im Wachstumsalter als ein Mittel zur Steigerung der geistigen und körperlichen Leistungsfähigkeit.

Die Wandschichten des Pollenkornes (Blütenstaub) sind jedoch so undurchlässig, dass auch der saure Magensaft, ebenso wie die übrigen Verdauungsorgane, nur bedingt dem Pollenkorn Inhaltsstoffe entnehmen können.

Gewöhnlich lassen sich in der Wandschicht des Pollenkornes zwei Schichten unterscheiden, die Exine oder äussere Wandschicht und die Intine, die innere Wandschicht. Bei der Exine unterscheidet man weiter eine skulpturierte Ektexine oder Sexine, und eine nicht skulpturierte Endexine oder Nexine. Mitunter scheint noch eine dritte Schicht, die Medine, dazwischenzuliegen.

Die Exine besteht hauptsächlich aus einem lipidartigen Stoff, dem Sporopollenin. Die Intine besteht im wesentlichen aus Pektinen, einem Gemisch von Polyuronsäuren und Polysacchariden.

Aufgrund der harten Schale der Pollenkörner ist es verständlich, dass der saure Magensaft sowie die übrigen Verdauungsorgane den Pollenkörnern verhältnismäßig wenig anhaben können und daher die Nutzung der in ihnen enthaltenen wertvollen Substanzen beschränkt ist. Ausserdem kann davon ausgegangen werden, dass die Resorption von Pollenteilen (etwa in der Grössenordnung von ca. 20 μm) in der Regel ca. 0,1 % beträgt. Dabei ist es noch anzunehmen, dass die Pollenteile eine elektrische Ladung aufweisen, die deren Resorption erst gewährleistet.

Durch die häufig vorgenommene Trocknung der gesammelten Pollenkörner, die ursprünglich über einen Feuchtigkeitsgehalt von ca. 15 % verfügen, erfährt die Pollenschale eine weitere Härtung und erweist sich gegenüber Verdauungssäften noch resistenter.

Bienenpollen dienen jedoch nicht nur zur Einnahme als leistungssteigerndes Naturprodukt, sie sind auch bei oraler Einnahme geeignet, eine Hyposensibilisierung gegen Pollinosen zu bewirken. Wenn ein Allergenträger, z. B. Blütenpollen, über längere Zeit über den Magen/Darmtrakt aufgenommen wird, gelangt ein geringer Teil der Allergene in die im oberen Darmtrakt zahlreich vertretenen Lymphknoten und wird dort verarbeitet. Man spricht von der sogenannten « low-dose »-Hyposensibilisierung. Es handelt sich um das modifizierte Sulzberger-Chase-Prinzip, wonach es unmöglich ist, ein Individuum zu allergisieren, wenn das entsprechende Allergen gleichzeitig oder vorausgehend über den Magen/Darmtrakt zugeführt wird.

Bei Kranken, die z. B. an Allergien gegen Blüten- und Gräserstaub, z. B. Heuschnupfen, Heufieber, Heuasthma, Pollen-Konjunctivis leiden, liegt eine Pollenallergie bereits vor. Klinische Versuche haben gezeigt, dass durch die lang dauernde und niedrig dosierte Zufuhr der entsprechenden Allergene eine Hyposensibilisierung und damit Heilung der Pollenkrankheit erreicht werden kann.

Bei der Verwendung von Bienenpollen zur Hyposensibilisierung von an Pollinosen Erkrankten ist es ebenfalls erforderlich, dass die Schale der Pollen im Magen/Darmtrakt soweit aufgeschlossen ist, dass eine Verwertung der Allergene möglich ist. Ausserdem ist es hier von Bedeutung, dass dem Patienten zur Hyposensibilisierung ein Pollengemisch zur Verfügung steht, welches mit grösster Wahrscheinlichkeit die Allergene sämtlicher in einem bestimmten Biotop auftretenden Blütenfamilien enthält, und damit mit grosser Sicherheit auch das zur Heilung der Pollinose erforderliche Antigen zur Verfügung stellt.

In diesem Zusammenhang ist es von Interesse, dass z. B. die im mitteleuropäischen Raum gesammelten Pollenkörner eine Vielzahl von Pollenarten aufweisen. Im Gegensatz dazu findet man in Ländern mit Grossanbauflächen und klimatisch bevorzugten Gebieten aufgrund der vorherrschenden Monokulturen nur Pollenkörner von weit weniger verschiedenen Pollenarten.

Es sind bereits verschiedene Methoden bekannt, um die Inhaltsstoffe von Pollen für die Desensibilisierung von Allergien nutzbar zu machen. Dabei handelt es sich ausschliesslich um Extraktionsverfahren, wobei mit Wasser und/bzw. fettlösenden Solventien die Pollen extrahiert werden. So beschreibt z. B. die DE-AS 11 40 671 die Extraktion von Pollen mit einem wässrigen und einem schwachbasischen tertiären heterocyclischen Amin für die subkutane Injektion zur prophylaktischen und therapeutischen Behandlung von Allergien. Da die vorzugsweise parenteral injizierbaren Allergenpräparate das Risiko von anaphylaktischen Reaktionen tragen, wurden verschiedene Verfahren entwickelt, um die Reaktion des Allergens abzuschwächen. Diese Verfahren sehen z. B. eine Vernetzung mit Cyanat, eine oxidativen Behandlung, oder einer Behandlung mit Aldehyd vor. Diese Verfahren werden z. B. in der DE-AS 20 30 223, in der DE-OS 29 32 604 und DE-OS 29 39 557 beschrieben.

Aufgabe der vorliegenden Erfindung ist es, ein Verfahren zur Verfügung zu stellen, mit welchem es möglich ist, die Resorbierbarkeit der Bienenpollenkörner zu verbessern, so dass die Nutzung der in den Pollen enthaltenen wertvollen Substanzen sowie Allergene optimiert wird. Ausserdem umfaßt die Erfindung die Verwendung des Bienenpollengemisches mit vorbesserter Resorbierbarkeit zur Hyposensibilisierung gegen Pollinosen zur Herstellung eines Arzneimittels.

Die vorstehende Aufgabe wird dadurch gelöst, daß das Bienenpollengemisch gemäß den Ansprüchen 1, 2, 5 und 6 autofermentativ, enzymatisch, mittels eines Gärungserregers oder mittels einer Säure behandelt wird.

Die « autofermentative » Behandlung der Bienenpollen erfolgt durch Behandlung des Bienenpollengemisches vorzugsweise in einem Brutschrank, ohne dass fermentative Erreger oder Enzyme zu dem Pollengemisch zugegeben werden.

Das Pollengemisch befindet sich in wässriger Suspension von z. B. 100 bis 400 mg Pollen/cm³ $H_2O$, vorzugsweise 300-350 mg Pollen/cm³ $H_2O$, bzw. enthält vorzugsweise 20 bis 25 % $H_2O$. Es wird im Brutschrank 3 Tage bis 3 Wochen, vorzugsweise 14 Tage, auf ca. 37 °C gehalten.

Sobald der pH-Wert des ursprünglich nahezu neutralen Pollengemisches 4,0 bis 4,4 beträgt, wird die Behandlung abgebrochen ; bei diesem pH-Wert kommt der Gärungsprozess zum Stillstand. Die Pollenkörner werden — sofern sie vorher mit einer grösseren Wassermenge vermischt wurden — durch Sedimentation oder Absetzenlassen und anschliessendes Abdekantieren des Überstandes vom wässrigen Medium weitgehend abgetrennt, wobei eine leicht bröckelige Paste mit einer Restfeuchtigkeit von 10 bis 20 %, vorzugsweise 12 bis 15 %, erhalten wird.

Der dekantierte Überstand wird gegebenenfalls eingeengt und der dabei erhaltene Rückstand der vorstehend erhaltenen Paste zugegeben. Auf diese Weise wird ein Gemisch aus Bienenpollen erhalten, das eine wesentlich verbesserte Resorbierbarkeit bei der Einnahme als Heil- und Aufbaustoff zeigt.

Wenn den Pollen vor dem Gärungsprozess nur ca. 20 % Wasser zugegeben worden waren, ist ein Sedimentieren oder Absetzenlassen nicht erforderlich, da die erhaltene Masse ohnehin pastöser Natur ist.

Es wird angenommen, dass die autofermentative Gärung durch solche Gärungserreger oder Fermente bewirkt wird, welche den von der Biene gesammelten Pollen anhaften. So kann z. B. Nectaromyces als Gärungserreger wirken und nach Durchführung des erfindungsgemässen Verfahrens zu einem Bienenpollengemisch mit verbesserter Resorbierbarkeit führen.

Durch die vorstehend beschriebene « autofermentative » Behandlung der Bienenpollen werden die in diesen enthaltenen Antigene nicht geschädigt, wohl aber die Verwertbarkeit der in den Pollen enthaltenen Vitalstoffe verbessert. Insbesondere bleiben bei dem Gärungsprozess die antigene Wirkung sowie auch die Vitamine, besonders Vitamin K, erhalten.

Eine bessere Resorbierbarkeit des Pollengemisches im Magen/Darmtrakt kan auch erzielt werden durch Behandlung im sauren Milieu. Es konnte beobachtet werden, dass die Pollen in Gegenwart einer anorganischen bzw. organischen Säure im Bereich der Keimpore stark anschwellen. Dieses Anschwellen setzt bereits innerhalb weniger Minuten in Gegenwart von Säure ein. Während die äussere Struktur der Porenwandung beibehalten wird, bedingt das starke Anschwellen der Pollen im Bereich der Keimpore eine leichtere Zugängigkeit zum Zellinhalt.

Zur Behandlung im sauren Milieu verwendet man bevorzugt Phosphorsäure und Essigsäure. Insbesondere ist es bevorzugt, diese Säuren zusammen mit einem Salz derselben einzusetzen und dadurch ein gepuffertes Milieu im pH-Bereich von 3,0 bis 5,0, bevorzugt ca. 4,0, zu schaffen. Die Behandlung wird in Abhängigkeit vom pH-Wert 6 h bis 3 Tage bei 30 bis 37 °C durchgeführt. Im weiteren ist auch der. Einsatz von Schwefelsäure geeignet.

Es hat sich gezeigt, dass durch das starke Anschwellen des Pollengemisches, dieses im Magen/Darmtrakt leichter zugängig ist und eine bessere Resorption erfährt.

Nach einer weiteren Modifikation des erfindungsgemässen Verfahrens kann eine verbesserte Resorbierbarkeit von Bienenpollen dadurch erreicht werden, dass die Pollen fermentativ behandelt werden. Zu diesem Zweck arbeitet man analog dem vorstehend für die autofermentative Behandlung beschriebenen Verfahren, wobei jedoch dem Pollengemisch das sich in einer wässrigen Suspension befindet, mindestens jedoch 20 bis 25 % $H_2O$ enthält, geeignete Gärungserreger zugegeben werden.

Unter den geeigneten Gärungserregern sind insbesondere zu nennen : Milchsäurebakterien und Gärhefen, insbesondere Nectaromyces.

Dabei hat es sich als besonders vorteilhaft erwiesen, eine vorbehandelte wässrige Lösung von hefereichem Honig, wie z. B. Löwenzahnhonig, mit dem Pollengemisch innig zu vermischen und dem erfindungsgemässen Gärverfahren zu unterziehen.

Die vorstehend erwähnte Vorbehandlung besteht z. B. darin, dass ein Wasser/Honig-Gemisch von ca. 1 : 0,2 bis 1 : 2 auf erhöhter Temperatur, d. h. 60-80 °C, 5 Minuten lang gehalten wird. Es wird angenommen, dass durch die Wärmebehandlung solche Gärungserreger bzw. Fermente denaturiert werden, die den Gärungsprozess nachteilig beeinflussen, z. B. indem sie die Gärmasse zu stark aufbläche lassen würden.

Der Gärvorgang kann in Anwesenheit oder Abwesenheit von Sauerstoff ablaufen.

Je nach dem verwendeten Gärungserreger werden die Bienenpollen einer Milchsäuregärung oder einer Hefegärung unterzogen.

Bei Verwendung eines Pollengemisches mit einem verhältnismässig geringen Wasserzusatz (ca. 20-30 %) wird das Wasser durch Aufquellen der Pollenkörner absorbiert. Es erübrigt sich damit ein Abtrennen von Flüssigkeit.

Zur schnelleren Ingangsetzung der Gärung ist es aber auch vorteilhaft, vom vorhergehenden Ansatz auf den neuen überzuimpfen.

Gemäss einer weiteren Modifizierung des erfindungsgemässen Verfahrens wird der von Bienen gesammelte Pollen einer enzymatischen Vorbehandlung unterzogen. Dabei ist es erforderlich, dem Bienenpollengemisch, welches sich in wässriger Suspension befindet, mindestens jedoch 20 bis 25 % $H_2O$ enthält, ein Enzym bzw. Enzyme zugegeben, welche auf die in den Wandschichten enthaltenen Substanzen, wie Lipide, Sporopollenin, Polyuronsäuren, Polysaccharide, Cellulose, einzuwirken vermögen. Als besonders geeignete Enzyme haben sich dabei Lactatdehydrogenase Cellulase, Lipase und Proteasen erwiesen. Ausserdem konnten gute Ergebnisse im Hinblick auf die Resorbierbarkeit des behandelten Pollengemisches bei Zugabe von Pectinase und β-1,3-Glucanase erhalten werden. Die Zugabe dieser Enzyme zum Pollengemisch führt zu einer schnelleren Pollenschlauchbildung. Es wird eine Schwellung des Pollengemisches beobachtet. Dabei werden die Enzyme in einer Konzentration zugegeben, die geeignet ist, um über einen Zeitraum von 1 bis 14 Tagen eine ausreichende Einwirkung auf die Wandschichten der Pollenkörner zu gewährleisten. Die Enzymkonzentration wird im allgemeinen 0,01 mg/ml bis 0,5 mg/ml betragen. Das enzymhaltige Pollengemisch bzw. Suspension wird dazu auf einer Temperatur von vorzugsweise 20 bis 37 °C gehalten.

Ausserdem ist bei der fermentativen Behandlung der Bienenpollenkörner das pH-Optimum der Aktivität des eingesetzten Enzyms zu beachten. Aus diesem Grunde kann es zweckmässig sein, dem Bienenpollengemisch eine vom ernährungsphysiologischen Standpunkt geeignete Pufferlösung zuzugeben.

Bei Verwendung von Proteasen, wie Chymotropsin oder Trypsin, ist es z. B. vorteilhaft, das enzymatisch zu behandelnde Gemisch auf pH 7-8 zu puffern. Mit Lipase zu behandelnde Gemische werden vorteilhaft auf pH 7-9 gepuffert. Das pH-Optimum der Lactatdehydrogenaseaktivität liegt bei ca. pH 7,0, so dass sich hier eine Einstellung des pH-Wertes, gegebenenfalls in Anwesenheit eines geeigneten Puffers, auf pH 6-8, empfiehlt.

Bei Zugabe geringer $H_2O$- oder Puffermengen werden diese bei der enzymatischen Behandlung von den Pollen absorbiert. Es erübrigt sich ein Abtrennen von Flüssigkeit.

Um zu vermeiden, dass nach Abschluss des erfindungsgemässen Verfahrens die Paste der vorbehandelten Bienenpollen eine Nachreaktion bzw. Nachgärung erfährt, wird diese in einem pflanzlichen Öl, welches einen höheren Anteil ungesättigter Fettsäuren aufweist, resuspendiert und homogenisiert. Als besonders vorteilhaft hat sich hierzu z. B. Sojaöl erwiesen.

Anstelle der vorstehenden Massnahme bzw. in Kombination mit derselben ist es auch möglich, der Pollenpaste zur Verhinderung einer Nachreaktion bzw. Nachgärung Harnstoff in einer Menge von 50 bis 100 mg/ml zuzugeben. Die Harnstoffzugabe dient sowohl der Konservierung und stellt ausserdem eine die Resorption fördernde Substanz dar, d. h. die Zugabe von Harnstoff fördert die Utilisierung der Pollenkörner im Darmtrakt (Duodenum, oberes Jejunum).

Sofern die Paste in Gelatinekapseln abgefüllt werden soll, kann dieser zur Vermeidung von Klumpenbildung Calciumcarbonat in einer Menge von 20 mg/ml bis 50 mg/ml zugegeben werden.

Mit Hilfe des erfindungsgemässen Verfahrens zur Verbesserung der Resorbierbarkeit wird ein Bienenpollengemisch erhalten, welches in Abhängigkeit von der Flora eines bestimmten Landstriches einen definierten Proteingehalt aufweist.

Das im Raum Mitteleuropa erhaltene Pollengemisch enthält in der Regel Pollenkörner der wichtigsten Blütenarten, wobei Bienenpollenkörner im allgemeinen nur ca. 8 bis 10 % Gräserpollen enthalten. Dem Bienenpollengemisch gibt man vorzugsweise Gräser- und Roggenpollen bis auf 20 % zu. Man kann auf diese Weise in bezug auf Zusammensetzung und Gehalt in Abhängigkeit von der Jahreszeit ein weitgehend standardisiertes Pollengemisch erhalten. Für die Hyposensibilisierung gegen Pollinosen verwendet man bevorzugt ein Pollengemisch, das als Leitpollen zumindest jeweils eine Pollenart aus der Gräsergruppe, der Bäumegruppe, der Gruppe der Wiesenblumen und der Gruppe der Frühblüher enthält. Auf diese Weise ist es möglich, einen Grossteil der in der Zeit von März bis Ende August auftretenden Allergien zu überdecken.

Wie bereits vorstehend ausgeführt, enthält ein beliebiges Biotop, das ist der Lebensraum von Menschen, Tieren und Pflanzen, eine Vielzahl heimischer Kräuter-, Gräser-, Bäume- und Getreidepollen. Da in einem bestimmten Biotop der in diesem Raum auftretenden Pollen beim Menschen zu Allergien führen kann, ist es zur Hyposensibilisierung gegen Pollinosen erforderlich, dass das hier zu verwendende Pollengemisch Pollen der wichtigsten Blütenarten aufweist.

Im Gegensatz dazu weisen Pollengemische, welche in Gegenden mit Monokulturen gesammelt werden, nur wenig verschiedene Pollenarten auf. Ein derartiges Bienenpollengemisch mit einer geringen Anzahl von Pollenarten wäre nach entsprechender Vorbehandlung zwar zur Hyposensibilisierung der in diesem Biotop auftretenden Allergien geeignet, es könnte jedoch nur mit geringerer Erfolgswahrscheinlichkeit zur Hyposensibilisierung von Pollinosen verwendet werden, die in einem Gebiet mit einer grösseren Anzahl heimischer Pflanzenfamilien auftreten.

Zur Hyposensibilisierung von Pollinosen ist es deshalb erforderlich, ein Pollengemisch einzusetzen, welches ein Spektrum der in dem entsprechenden Biotop von Bienen üblicherweise gesammelten Pollen aufweist und ausserdem dem Biotop entstammt, in dem auch die entsprechenden Pollinosen ihren Ursprung haben.

Die erfindungsgemäss vorbehandelten Bienenpollen weisen folgende Vorteile auf :
a) Sie sind im allgemeinen gut verträglich.
b) Die Wirksamkeit der in den Pollen enthaltenen Vitalstoffe ist verstärkt.
c) Die Einnahme erfindungsgemäss vorbehandelter Pollen regt offensichtlich die Darmtätigkeit an.
d) Erfindungsgemäss vorbehandelte Pollen steigern in besonderem Masse die geistige und körperliche Leistungsfähigkeit.

Beispiel 1

Behandlung eines Bienenpollengemisches durch Zugabe von Gärungserregern

15 Gewichtsteile Löwenzahn-Honig werden mit 25 Gewichtsteilen Wasser vermischt. Die erhaltene Lösung wird 5 bis 10 Minuten lang auf eine Temperatur von 65 bis 70 °C erwärmt.

Nach dem Abkühlen gibt man diese zu 100 Gewichtsteilen eines Bienenpollengemisches. Die dabei erhaltene Masse hält man 5 bis 12 Tage lang im Brutschrank auf einer Temperatur von 35-36 °C. Dabei ist es vorteilhaft, die Masse z. B. mit einem Holzbrett zu beschweren. Man beobachtet die Masse, welche sich im Verlauf des Gärprozesses immer mehr ausdehnt. Diese Ausdehnung der Pollenmasse kommt nach Beendigung des Gärprozesses zum Stillstand. Sobald keine Ausdehnung der Pollenmasse mehr zu beobachten ist, entnimmt man dieselbe dem Brutschrank und bewahrt sie bei Raumtemperatur oder im Kühlschrank luftdicht auf. Das erhaltene Pollengemisch ist über einen längeren Zeitraum hinweg gut haltbar.

Das fermentativ behandelte Pollengemisch weist gegenüber einem unbehandelten eine wesentlich verbesserte Resorbierbarkeit durch den Magen/Darm-Trakt auf.

Beispiel 2

Behandlung eines Pollengemisches im sauren Milieu

Zu 100 Gewichtsteilen eines Bienenpollengemisches gibt man 50 Gewichtsteile einer 0,15 molaren Lösung aus Phosphorsäure und Natriumphosphat mit einem pH-Wert von 3,5. Man rührt das Gemisch gründlich und lässt dieses 24 h im Brutschrank auf einer Temperatur von 30 °C. Es wird ein Anschwellen der Pollenmasse beobachtet.

Nach der Behandlung wird die feuchte Pollenmasse mit einem pflanzlichen Öl, welches einen höheren Anteil ungesättigter Fettsäuren aufweist, suspendiert und homogenisiert.

Beispiel 3

Enzymatische Behandlung eines Bienenpollengemisches

Zu 100 Gewichtsteilen eines Bienenpollengemisches gibt man 300 Gewichtsteile 0,15 m Phosphatpuffer, pH 7,0, welcher die Enzyme Pectinase (0,5 mg/ml), Cellulase (0,5 mg/ml) und $\beta$-1,3-Glucanase (0,5 mg/ml) enthält.

Man lässt das Gemisch 2 Tage lang bei 30 °C im Brutschrank stehen. Es wird hier ebenfalls ein Aufquellen der vom wässrigen Überstand befreiten Pollenmasse beobachtet. Der Rückstand wird lyophilisiert und zur Pollenmasse gegeben.

Nach der Behandlung im Brutschrank mischt man dem Pollengemisch 100 Gewichtsteile eines pflanzlichen Öls, welches einen höheren Gehalt an ungesättigten Fettsäuren aufweist, zu.

Das derart behandelte Pollengemisch zeichnet sich durch eine deutlich verbesserte Resorbierbarkeit im Magen/Darmtrakt aus.

**Patentansprüche**

1. Verfahren zu Herstellung eines als Heilmittel und Aufbaustoff dienenden Bienenpollengemisches mit verbesserter Resorbierbarkeit, dadurch gekennzeichnet, daß
a) das Bienenpollengemisch in wäßriger Suspension, mindestens aber in Gegenwart von ca. 20 bis 25 % Wasser, 3 Tage bis 3 Wochen auf ca. 37 °C gehalten wird,
b) bei Einstellung eines pH-Wertes von 4,0 bis 4,4 die Behandlung unter a) abgebrochen wird,
c) die Pollen gegebenenfalls durch Sedimentation oder Absetzenlassen und anschließendes Abdekantieren des Überstandes von wäßrigem Medium weitgehend abgetrennt werden, wobei eine leicht bröckelige Paste mit einem Restfeuchtigkeitsgehalt von 10 bis 20 %, vorzugsweise 12 bis 15 %, erhalten wird,
d) der Überstand gegebenenfalls im Vakuum eingeengt und der Rückstand zu der unter c) erhaltenen Paste zugegeben wird.
2. Verfahren zur Herstellung eines als Heilmittel und Aufbaustoff dienenden Bienenpollengemisches mit verbesserter Resorbierbarkeit, dadurch gekennzeichnet, daß das Pollengemisch in wäßriger Suspension, mindestens aber in Gegenwart von 20 bis 25 % $H_2O$ unter Zusatz eines geeigneten Gärungserregers, vorzugsweise Milchsäurebakterien oder Gärhefen,

a) 3 Tage bis 3 Wochen auf ca. 37 °C gehalten,

b) bei Einstellung eines pH-Wertes von 4,0 bis 4,4 die Behandlung unter a) abgebrochen wird,

c) die Pollen gegebenenfalls durch Sedimentation oder Absetzenlassen und anschließendes Abdekantieren des Überstandes vom wäßrigen Medium weitgehend abgetrennt werden, wobei eine leicht bröckelige Paste mit einem Restfeuchtigkeitsgehalt von 10 bis 20 % erhalten wird,

d) der Überstand gegebenenfalls im Vakuum eingeengt und der Rückstand zu der unter c) erhaltenen Paste zugegeben wird.

3. Verfahren nach Anspruch 2, dadurch gekennzeichnet, daß als Gärungserreger Nectaromyces zugesetzt wird.

4. Verfahren gemäß Ansprüchen 1 bis 3, dadurch gekennzeichnet, daß zur schnelleren Ingangsetzung der Gärung von der alten Maische auf die neue übergeimpft wird.

5. Verfahren zur Herstellung eines als Heilmittel und Aufbaustoff dienenden Bienenpollengemisches mit verbesserter Resorbierbarkeit, dadurch gekennzeichnet, daß

a) das Pollengemisch in wäßriger, gegebenenfalls gepufferter Suspension, mindestens jedoch in Gegenwart von 20 bis 25 % $H_2O$ oder Pufferlösung unter Zusatz eines oder mehrerer Enzyme aus der Gruppe Lactatdehydrogenase, Cellulase, Proteinasen und Lipase, 1 bis 10 Tage lang auf 20 bis 37 °C gehalten wird,

b) das Pollengemisch gegebenenfalls durch Sedimentation oder Absetzenlassen und anschließendes Abdekantieren des Überstandes vom wäßrigen Medium weitgehend abgetrennt wird, wobei eine leicht bröckelige Past mit einem Restfeuchtigkeitsgehalt von 10 bis 20 % erhalten wird,

c) der Überstand gegebenenfalls im Vakuum eingeengt und der Rückstand zu der unter b) erhaltenen Paste zugegeben wird.

6. Verfahren zur Herstellung eines als Heilmittel und Aufbaustoff dienenden Bienenpollengemisches mit verbesserter Resorbierbarkeit, dadurch gekennzeichnet, daß

a) das Bienenpollengemisch in wäßriger Suspension, mindestens aber in Gegenwart von ca. 20 bis 25 % Wasser, durch Zugabe einer geeigneten anorganischen oder organischen Säure auf pH 3,0 bis 5,0 gebracht und 3 Tage bis 3 Wochen auf ca. 37 °C belassen wird,

b) der Pollen vom wäßrigen Medium weitgehend abgetrennt wird.

7. Verfahren gemäß einem oder mehreren der vorangehenden Ansprüche, dadurch gekennzeichnet, daß die Paste zur Verhinderung einer Nachreaktion bzw. Nachgärung in einem pflanzlichen Öl, welches einen höheren Anteil ungesättigter Fettsäuren aufweist, resuspendiert und homogenisiert wird.

8. Verfahren nach einem oder mehreren der vorangehenden Ansprüche, dadurch gekennzeichnet, daß der erhaltenen Paste Harnstoff in einer Menge von 50-100 mg/ml zugegeben wird.

9. Verfahren nach einem oder mehreren der vorangehenden Ansprüche, dadurch gekennzeichnet, daß der Paste zur Vermeidung der Klumpenbildung Calciumcarbonat zugegeben wird.

10. Verfahren nach einem oder mehreren der vorangehenden Ansprüche, dadurch gekennzeichnet, daß als Ausgangspollen ein Pollengemisch eingesetzt wird, das von Bienen in dem Biotop gesammelt wurde, in welchem die auftretenden Pollinosen behandelt werden sollen.

11. Verwendung des Bienenpollengemisches mit verbesserter Resorbierbarkeit zur Hyposensibilisierung gemäß den voranstehenden Ansprüchen 1 bis 10, zur Herstellung eines Arzneimittels.

**Claims**

1. A process for preparing a mixture of bee pollen having an improved resorption capacity, said mixture of bee pollen serving as a medicament and restorative substance, characterized in that

a) the mixture of bee pollen is kept for 3 days up to 3 weeks at about 37 °C in aqueous suspension, however at least in the presence of about 20 to 25 % water,

b) the treatment under a) is terminated by adjusting the pH to the range from 4.0 to 4.4,

c) the pollen is essentially separated from the aqueous medium optionally by sedimentation or precipitation and subsequent decanting of the supernatant, thereby obtaining a slightly brittle paste with a content of residual humidity from 10 to 20 %, preferably 12 to 15 %,

d) the supernatant is optionally concentrated under vacuum and the residue is added to the paste obtained under c).

2. A process for preparing a mixture of bee pollen having an improved resorption capacity, said mixture of bee pollen serving as a medicament and restorative substance, characterized in that the mixture of pollen is kept in aqueous suspension, however, at least in the presence of 20 to 25 % $H_2O$, under addition of a suitable fermenting substance, preferably lactic acid bacteria of fermenting yeasts,

a) for 3 days up to 3 weeks at a temperature of about 37 °C,

b) the treatment under a) is terminated by adjusting the pH to the range of 4.0 to 4.4,

c) the pollen is essentially separated from the aqueous medium optionally by sedimentation or precipitation and subsequent decanting of the supernatant, thereby obtaining a slightly brittle paste with a content of residual humidity of 10 to 20 %,

d) the supernatant is optionally concentrated under vacuum and the residue is added to the paste obtained under c).

3. A process according to claim 2, characterized in that Nectaromyces is added as fermenting substance.

4. A process according to claims 1 to 3, characterized in that there is carried out an inocculation from the old to the new mash in order to accelerate fermentation.

5. A process for preparing a mixture of bee pollen having an improved resorption capacity, said mixture of bee pollen serving as a medicament and restorative substance, characterized in that

a) said mixture of pollen is kept one to ten days at 20 to 37 °C in an aqueous, optionally buffered suspension, however, at least in the presence of 20 to 25 % $H_2O$ or buffer solution, under addition of one or more enzymes selected from the group lactate dehydrogenase, cellulase, proteinases and lipase,

b) the mixture of pollen is essentially separated from the aqueous medium optionally by sedimentation or precipitation and subsequent decanting of the supernatant, thereby obtaining a slightly brittle paste with a content of residual humidity from 10 to 20 %,

c) the supernatant is optionally concentrated under vacuum and the residue is added to the paste obtained under b).

6. A process for preparing a mixture of bee pollen having an improved resorption capacity, said mixture of bee pollen serving as a medicament and restorative substance, characterized in that

a) said mixture of pollen is brought in aqueous suspension, however, at least in the presence of about 20 to 25 % water, to pH 3.0 to 5.0 by adding a suitable inorganic or organic acid and kept 3 days up to 3 weeks at about 37 °C,

b) the pollen is substantially separated from the aqueous medium.

7. A process according to one or more of the preceding claims, characterized in that the paste is resuspended and homogenized in a vegetable oil containing an increased amount of unsaturated fatty acids in order to prevent any post-reaction or post-fermentation.

8. A process according to one or more of the preceding claims, characterized in that urea in an amount from 50 to 100 mg/ml is added to the paste.

9. A process according to one or more of the preceding claims, characterized in that calcium carbonate is added to the paste in order to prevent formation of lumps.

10. A process according to one or more of the preceding claims, characterized in that a mixture of pollen is used as initial pollen which has been collected by bees in the biotrope where the occurring pollinoses are to be treated.

11. The use of a hyposensibilizing mixture of bee pollen with improved resorption capacity according to preceding claims 1 to 10 for the preparation of a pharmaceutical.

**Revendications**

1. Procédé de préparation d'un mélange de pollens d'abeilles, à capacité de résorption améliorée, servant comme médicament et comme élément reconstituant, caractérisé en ce que :

a) le mélange de pollens d'abeilles est maintenu à environ 37 °C, pendant 3 jours à 3 semaines, dans une suspension aqueuse, mais au moins en présence d'environ 20 à 25 % d'eau ;

b) le traitement indiqué en a) est interrompu par ajustement du pH à une valeur comprise entre 4,0 et 4,4 ;

c) on sépare complètement les pollens du milieu aqueux, éventuellement par sédimentation ou en les laissant se déposer, et en séparant ultérieurement par décantation le surnageant, ce par quoi l'on obtient une pâte facilement friable présentant une teneur résiduelle en humidité de 10 à 20 %, de préférence de 12 à 15 % ;

d) le surnageant est éventuellement concentré sous vide, et le résidu ajouté à la pâte obtenue en c).

2. Procédé de préparation d'un mélange de pollens d'abeilles, servant de médicament et d'élément reconstituant, à capacité de résorption améliorée, caractérisé en ce que :

a) le mélange de pollens est maintenu pendant 3 jours à 3 semaines, à environ 37 °C, en suspension aqueuse, mais au moins en présence de 20 à 25 % d'eau, avec addition d'un ferment approprié, de préférence des ferments lactiques ou des levures de fermentation ;

b) le traitement indiqué en a) est interrompu par ajustement du pH à une valeur comprise entre 4,0 et 4,4 ;

c) on sépare complètement les pollens du milieu aqueux, éventuellement par sédimentation ou en les laissant se déposer, et par décantation ultérieure du surnageant, ce par quoi on obtient une pâte facilement friable présentant une teneur résiduelle en humidité de 10 à 20 % ;

d) le surnageant est éventuellement concentré sous vide, et le résidu ajouté à la pâte obtenue en c).

3. Procédé selon la revendication 2, caractérisé en ce que l'on ajoute, en tant que ferments, des Nectaromyces.

4. Procédé selon les revendications 1 à 3, caractérisé en ce que, pour la mise en œuvre plus rapide de la fermentation, on inocule le nouveau moût avec du vieux.

5. Procédé de préparation d'un mélange de pollens d'abeilles, servant comme médicament et comme élément reconstituant, à capacité de résorption améliorée, caractérisé en ce que :

a) le mélange de pollens est maintenu pendant 1 à 10 jours, à 20-37 °C, en suspension aqueuse éventuellement tamponnée, mais cependant en présence de 20 à 25 % d'eau ou de solution tampon, avec addition d'une ou plusieurs enzymes du groupe constitué par la lactate déhydrogénase, la cellulase, les protéinases, et la lipase ;

b) on sépare complètement le mélange de pollens du milieu aqueux, éventuellement par sédimentation ou en le laissant se déposer, et en séparant ultérieurement le surnageant par décantation, ce par quoi on obtient une pâte facilement friable, présentant une teneur résiduelle en humidité de 10 à 20 % ;

c) le surnageant est éventuellement concentré sous vide, et le résidu ajouté à la pâte obtenue en b).

6. Procédé de préparation d'un mélange de pollens d'abeilles, servant comme médicament et élément reconstituant, à capacité de résorption améliorée, caractérisé en ce que :

a) le mélange de pollens d'abeilles, en suspension aqueuse, mais au moins en présence d'environ 20 à 25 % d'eau, est amené à pH 3,0 à 5,0 par addition d'un acide inorganique ou organique approprié, et abandonné à environ 37 °C pendant 3 jours à 3 semaines ;

b) le pollen est séparé complètement du milieu aqueux.

7. Procédé selon une ou plusieurs des revendications précédentes, caractérisé en ce que, pour empêcher une réaction ou une fermentation ultérieure, on remet la pâte en suspension dans une huile végétale, présentant une proportion élevée d'acides gras insaturés, et on homogénéise.

8. Procédé selon une ou plusieurs des revendications précédentes, caractérisé en ce que l'on ajoute à la pâte obtenue de l'urée en une quantité de 50 à 100 mg/ml.

9. Procédé selon une ou plusieurs des revendications précédentes, caractérisé en ce que l'on ajoute à la pâte du carbonate de calcium pour éviter la formation de grumeaux.

10. Procédé selon une ou plusieurs des revendications précédentes, caractérisé en ce que l'on utilise, comme pollen de départ, un mélange de pollens recueillis sur des fleurs présentes dans le biotope dans lequel on doit traiter les pollinoses qui apparaissent.

11. Utilisation du mélange de pollens de fleurs à capacité de résorption améliorée, pour l'hyposensibilisation, selon les revendications précédentes 1 à 10, pour la préparation d'un produit pharmaceutique.